Europäisches Patentamt

European Patent Office

Office européen des brevets

(11) Veröffentlichungsnummer: **0 106 115**
**A2**

(12) **EUROPÄISCHE PATENTANMELDUNG**

(21) Anmeldenummer: 83108807.5

(22) Anmeldetag: 07.09.83

(51) Int. Cl.³: **C 07 J 51/00**

(30) Priorität: 10.09.82 DE 3233602

(43) Veröffentlichungstag der Anmeldung:
25.04.84 Patentblatt 84/17

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Harder, Hans-Joachim, Dr.
Eppenhainer Strasse 6
D-6233 Kelkheim (Taunus)(DE)

(72) Erfinder: Stache, Ulrich, Dr.
Goldgrabenstrasse 20
D-6238 Hofheim Taunus(DE)

(72) Erfinder: Haede, Werner, Dr.
Herderstrasse 15
D-6238 Hofheim am Taunus(DE)

(54) Verfahren zur Reinigung von Corticosteroidphosphorsäureester-Alkalisalzen und ihre Verwendung.

(57) Es wird ein Vefahren zur Reinigung von Corticosteroid-phosphorsäureester-Alkalisalzen mit Hilfe von Aktivkohle beschrieben. Die hochgereinigten Verbindungen eignen sich zur Herstellung von klarstabilen Lösungen.

EP 0 106 115 A2

0106115

HOECHST AKTIENGESELLSCHAFT    HOE 82/F 177        Dr.D/Pa

## Verfahren zur Reinigung von Corticosteroidphosphorsäure-ester-Alkalisalzen und ihre Verwendung

Die Erfindung betrifft ein Verfahren zur Reinigung von Corticosteroidphosphorsäureester-Alkalisalzen und die Verwendung der so gereinigten Verbindungen zur Herstellung klarstabiler Lösungen.

Wäßrige Lösungen von Alkalisalzen der Corticosteroidphosphorsäureester können, insbesondere bei Temperaturbelastung, nach kurzer Zeit Trübungen aufweisen.

Das Auftreten von Trübungen dieser Lösungen bzw. Ausflockungen von z.B. Zersetzungsprodukten des Wirkstoffs, kann durch geeignete technische Maßnahmen, wie z.B. Wahl eines geeigneten pH-Wertes oder Zusatz eines Oxydationsschutz,wie z.B. Antioxidantien, verzögert,jedoch nicht ausgeschlossen werden.

Der Zusatz geeigneter Lösungsmittel, wie 1,3-Butandiol, kann ebenfalls das Auftreten von Trübungen verzögern. Solche Lösungsmittel sind aber aus toxikologischen Gründen unerwünscht, und ihr Effekt ist unsicher. Außerdem kann eine Zersetzung des Wirkstoffs unter Umständen nicht erkannt werden, da das Zersetzungsprodukt in dem zugesetzten Lösungsmittel löslich sein kann.
Die Trübungen traten bei Lösungen, die man den erwähnten Maßnahmen unterworfen hatte, trotz eines hohen Reinheitsgrades der Alkalisalze der eingesetzten Corticosteroidphosphorsäureester, wie er unter den Bedingungen der in der Corticosteroidchemie üblichen Reinigungsschritte zu erhalten ist, auf. Der Reinheitsgrad wurde durch Hochdruckflüssigkeitschromatographie (reversed phase HPLC) und anderen üblichen Methoden bestimmt und lag bei etwa 97 % oder höher.

Trübe Lösungen ursprünglich klarer Lösungen sind für die medizinische Anwendung, insbesondere für Injektionszwecke, ungeeignet, da die Trübung vielfach auf Veränderungen des Wirkstoffs beruht. Darüberhinaus dürfen solche Lösungen nicht parenteral angewendet werden. Die genannten Corticosteroidderivate werden vielfach bei lebensbedrohlichen Zuständen i.v. appliziert. Hierbei ist es zweckmäßig, daß der Wirkstoff in spritzfertiger Lösung vorliegt und daß keine besonderen Lagerungshinweise und Verfallsdaten zu beachten sind.

Aufgabe der Erfindung war es daher, solche Corticosteroidphosphorsäureester-Alkalisalze zur Verfügung zu stellen, aus denen klarstabile Lösungen hergestellt werden können.

Überraschenderweise wurde gefunden, daß die Trübungen der Lösungen selbst bei Temperaturbelastung ausblieben, wenn ein zusätzlicher Reinigungsschritt für die Alkalisalze der Corticosteroidphosphorsäureester eingeführt wird. Dieser zusätzliche Reinigungsschritt besteht in einer Behandlung einer im wesentlichen wäßrigen Lösung der Alkalisalze der Corticosteroidphosphorsäureester mit Aktivkohle und Abtrennung der Aktivkohle z.B. durch ein Sterilfilter. Die Lösung enthält das hochgereinigte Corticosteroidphosphorsäureester-Alkalisalz. Sie kann sofort z.B. zu Injektionslösungen weiterverarbeitet oder auch einer Gefriertrocknung unterworfen werden.

Die Erfindung betrifft daher ein Verfahren zur Herstellung von hochgereinigten Corticosteroidphosphorsäure-Alkalisalzen sowie ihre Verwendung zur Herstellung klarstabiler Lösungen.

Das Verfahren ist dadurch gekennzeichnet, daß man eine im wesentlichen wäßrige Lösung eines Corticosteroidphosphorsäureester-Alkalisalzes mit Aktivkohle behandelt.

und anschließend die Aktivkohle abtrennt.

Die Abtrennung der Kohle erfolgt z.B. durch Abzentrifugieren, vorzugsweise durch Sterilfiltration. Die so erhaltenen Lösungen können sofort zu klarstabilen Lösungen wie z.B. Injektionslösungen verarbeitet werden, wobei z.B. auf einen gewünschten Wirkstoffgehalt und pH-Wert eingestellt und gegebenenfalls ein Puffer zugesetzt wird.

Vorzugsweise wird aus den Lösungen nach Abtrennung der Kohle das Lösungsmittel entfernt. Dies geschieht zweckmäßigerweise durch Gefriertrocknung. Aus dem so getrockneten, hochreinen Wirkstoff können durch Zusatz von Lösungsmitteln wie Wasser, Puffer, Antioxidantien klarstabile Lösungen hergestellt werden.

Als Aktivkohle eignet sich medizinische Kohle (z.B. entspr. DAB7), insbesondere eine solche mit einem Aschegehalt von $< 1,0$ Gew.%, einer überwiegenden Korngröße von $< 40$ μm und einer BET-Oberfläche von etwa 750 bis 850 $m^2/g$.

Als Corticosteroidphosphorsäureester kommen insbesondere Desoximetason-, Dexamethason-, Triamcinolon- und Methylprednisolon-dihydrogenphosphat in Betracht. Als Alkalisalze eignen sich die Natrium- und Kaliumsalze, insbesondere die Dinatrium- und Dikaliumsalze. Besonders bevorzugt ist 6-Methyl-prednisolon-21-dihydrogenphosphat-Dinatrium-Salz.

Die reversed phase HPLC-Chromatogramme der Alkalisalze der Corticosteroidphosphorsäureester wiesen vor und nach dem Reinigungsschritt keine wesentlichen Unterschiede auf. Die Klarstabilität der aus den hochgereinigten Verbindungen hergestellte Lösungen unterschied sich jedoch bei Temperaturbelastung überraschenderweise erheblich. Der durch den beanspruchten Reinigungsschritt erzielte Effekt erlaubt, auf den Zusatz von Lösungsmitteln wie 1,3-Butadiol zu verzichten. Der Zusatz von Puffern und Antioxidantien ist zweckmäßig.

Injektionslösungen, die ein nach dem erfindungsgemäßen

- 4 -

Verfahren gereinigtes 6-Methylprednisolon-21-dihydrogenphosphat-Dinatriumsalz enthielten, waren nach 3 monatiger
Belastung bei +40°C klar und zeigten keinerlei Trübung
oder Ausflockung.

Beispiel 1:

30 g 6-Methylprednisolon-21-dihydrogenphosphat-dinatriumsalz werden in 70 g gereinigtem Wasser gelöst. Die Lösung
wird mit 50 g Kohle (Charakterisierung s. Seite 3; Zeilen
16-18) versetzt und unter Lichtschutz und Inertbegasung
bei Raumtemperatur 30 Minuten gerührt. Danach wird die
Kohle durch Filtration durch ein Sterilfilter abgetrennt.
Die klare Lösung wird gefriergetrocknet.

Patentansprüche:

1. Verfahren zur Reinigung von Corticosteroidphosphorsäureester-Alkalisalzen, dadurch gekennzeichnet, daß man eine im wesentlichen wäßrige Lösung eines Corticosteroidphosphorsäureester-Alkalisalzes mit Aktivkohle behandelt und die Kohle anschließend abtrennt.

2. Verfahren nach Anspruch 1, dadurch gekennzeichnet, daß man nach Abtrennung der Aktivkohle das Lösungsmittel entfernt.

3. Verfahren nach Anspruch 1, dadurch gekennzeichnet, daß man als Aktivkohle eine solche mit einem Aschegehalt von $<1,0$ Gew.%, einer überwiegenden Korngröße von $< 40\mu m$ und einer BET-Oberfläche von etwa 750 bis 850 $m^2/g$ verwendet.

4. Verfahren nach Anspruch 1, dadurch gekennzeichnet, daß man 6-Methylprednisolon-21-dihydrogenphosphat-Dinatriumsalz einsetzt.

5. Verfahren nach Anspruch 1, dadurch gekennzeichnet, daß man eine im wesentlichen wäßrige Lösung von 6-Methylprednisolon-21-dihydrogenphosphat-Dinatriumsalz mit Aktivkohle behandelt, die Kohle durch Sterilfiltration abtrennt und die so erhaltene Lösung der Gefriertrocknung unterwirft.

6. Hochgereinigte Alkalisalze von Corticosteroidphosphorsäureestern erhalten nach einem der Ansprüche 1 bis 5.

7. Verwendung eines nach Ansprüchen 1 bis 5 hergestellten Corticosteroidphosphorsäure-Alkalisalzes zur Herstellung von klarstabilen Lösungen.

0106115

- 1 -    HOE 82/F 177

Patentansprüche für Österreich:

1. Verfahren zur Reinigung von Corticosteroidphosphorsäure-ester-Alkalisalzen, dadurch gekennzeichnet, daß man eine im wesentlichen wäßrige Lösung eines Corticosteroidphosphorsäureester-Alkalisalzes mit Aktivkohle behandelt und die Kohle anschließend abtrennt.

2. Verfahren nach Anspruch 1, dadurch gekennzeichnet, daß man nach Abtrennung der Aktivkohle das Lösungsmittel entfernt.

3. Verfahren nach Anspruch 1, dadurch gekennzeichnet, daß man als Aktivkohle eine solche mit einem Aschegehalt von $< 1,0$ Gew.-%, einer überwiegenden Korngröße von $< 40$ µm und einer BET-Oberfläche von etwa 750 bis 850 m²/g verwendet.

4. Verfahren nach Anspruch 1, dadurch gekennzeichnet, daß man 6-Methylprednisolon-21-dihydrogenphosphat-Dinatriumsalz einsetzt.

5. Verfahren nach Anspruch 1, dadurch gekennzeichnet, daß man eine im wesentlichen wäßrige Lösung von 6-Methylprednisolon-21-dihydrogenphosphat-Dinatriumsalz mit Aktivkohle behandelt, die Kohle durch Sterilfiltration abtrennt und die so erhaltene Lösung der Gefriertrocknung unterwirft.